(19) Europäisches Patentamt
European Patent Office
Office européen des brevets

(11) Veröffentlichungsnummer: **0 119 510**
**B1**

(12) # EUROPÄISCHE PATENTSCHRIFT

(45) Veröffentlichungstag der Patentschrift: **22.04.87**

(21) Anmeldenummer: **84101863.3**

(22) Anmeldetag: **22.02.84**

(51) Int. Cl.⁴: **C 07 D 401/06**, C 07 D 405/14, C 07 D 409/14, C 07 D 417/14, A 61 K 31/505

(54) **Pyridin-Pyrimidinon-Derivate, Verfahren zu ihrer Herstellung und diese Verbindungen enthaltende Arzneimittel.**

(30) Priorität: **22.02.83 DE 3306102**

(43) Veröffentlichungstag der Anmeldung: **26.09.84 Patentblatt 84/39**

(45) Bekanntmachung des Hinweises auf die Patenterteilung: **22.04.87 Patentblatt 87/17**

(84) Benannte Vertragsstaaten: **AT BE CH DE FR GB IT LI LU NL SE**

(56) Entgegenhaltungen:
**EP - A - 0 015 138**
**EP - A - 0 086 647**
**EP - A - 0 087 274**

(73) Patentinhaber: **LUDWIG HEUMANN & CO GMBH, Heideloffstrasse 18-28 Postfach 2260, D-8500 Nürnberg (DE)**

(72) Erfinder: **Schickaneder, Helmut, Dr., Dipl.-Chem., Moosäcker 25, D-8501 Eckental (DE)**
Erfinder: **Mörsdorf, Peter, Dr., Dipl.-Chem., Untere Bahnhofstrasse 16, D-8501 Cadolzburg (DE)**
Erfinder: **Postius, Stefan, Dr., Biochem., Unschlittplatz 1, D-8500 Nürnberg (DE)**
Erfinder: **Szelenyi, Istvan, Dr. med., Brahmsstrasse 16, D-8501 Schwaig (DE)**
Erfinder: **Herter, Rolf, Dr., Dipl.-Chem., Bayernstrasse 42, D-8540 Schwabach (DE)**
Erfinder: **Hansen, Herbert, Untere Bahnhofstrasse 16, D-8501 Cadolzburg (DE)**

(74) Vertreter: **Kraus, Walter, Dr. et al, Patentanwälte Kraus, Weisert & Partner Thomas-Wimmer-Ring 15, D-8000 München 22 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue Pyridin-Pyrimidinon-Derivate mit einer hohen selektiven Wirkung auf Histamin-$H_2$-Rezeptoren, Verfahren zu ihrer Herstellung und Arzneimittel, die diese Verbindungen enthalten.

Als Antiulcusmittel hat Cimetidin bereits Eingang in die Therapie gefunden. Die Halbwertszeit von Cimetidin ist jedoch verhältnismässig kurz. Aus EP-A-0 087 274 und EP-A-0 086 647 sind bestimmte Pyrimidonderivate mit $H_2$-antagonistischer Aktivität bekannt.

Der Erfindung liegt die Aufgabe zugrunde, neue Hemmstoffe für Histamin-$H_2$-Rezeptoren mit verbesserter und/oder länger anhaltender Wirksamkeit zur Verfügung zu stellen.

Diese Aufgabe wird erfindungsgemäss durch Pyridin-Pyrimidinon-Derivate der allgemeinen Formel I:

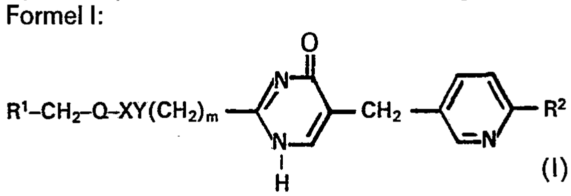

(I)

in der $R^1$ für Pyrrolidino oder Piperidino und $R^2$ für Wasserstoff oder Methyl stehen, Q für meta-Phenylen oder 2,4-Thiophendiyl steht, X im Fall, dass Q die Bedeutung m-Phenylen hat, für Sauerstoff, Y für eine Einfachbindung steht und m den Wert 4 hat, X im Fall, dass Q die Bedeutung 2,4-Thiophendiyl hat, für Methylen, Y für Schwefel steht und m den Wert 3 hat, sowie die physiologisch annehmbaren Salze davon gelöst.

Die erfindungsgemässen Verbindungen zeigen aufgrund ihrer spezifischen $H_2$-antagonistischen Aktivität eine Hemmung der Magensäuresekretion, wenn diese durch Histamin-Agonisten stimuliert wird [Ash und Schild, «Brit. J. Pharmacol. Chemother.», 27, 427 (1966) und Black u.a., «Nature», 236, 384 (1971)]. Weiterhin antagonisieren sie die Histaminwirkung auf die Kontraktionsfrequenz des isolierten rechten Atriums des Meerschweinchens, aber sie beeinflussen nicht histamininduzierte Kontraktionen des isolierten, glatten gastrointestinalen Muskels, wenn diese durch $H_2$-Agonisten hervorgerufen werden.

Nachdem Hemmstoffe für Histamin-$H_2$-Rezeptoren sowohl bezüglich der basalen Magensäuresekretion als auch der durch Gastrin, Histamin, Methacholin oder Nahrung induzierten Magensäuresekretion Hemmwirkung besitzen, können sie für die Behandlung von peptischen Ulcera, die durch übermässige Sekretion von Magensäure verursacht sind, und bei der Therapie hyperacider Gastritis verwendet werden.

Die erfindungsgemässen Pyridin-Pyrimidinon-Derivate können in folgenden tautomeren Formen

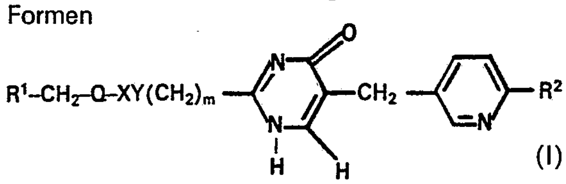

(I)

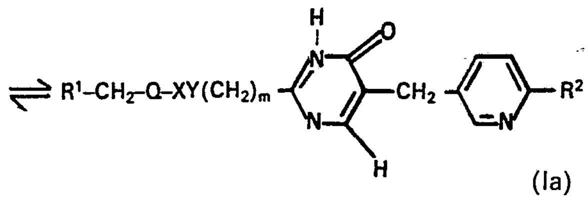

(Ia)

vorliegen, wobei $R^1$, $R^2$, Q, X, Y und m die oben angegebenen Bedeutungen besitzen.

Die erfindungsgemässen Verbindungen werden durch ein Verfahren hergestellt, das dadurch gekennzeichnet ist, dass man eine Verbindung der allgemeinen Formel II:

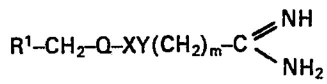

(II)

worin $R^1$, Q, X, Y und m die oben angegebenen Bedeutungen haben, in an sich bekannter Weise in basenkatalysierter Umsetzung mit einem Pyridinderivat der allgemeinen Formel III:

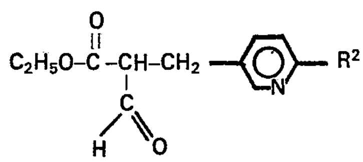

(III)

worin $R^2$ die oben angegebene Bedeutung hat, umsetzt und die erhaltene Verbindung gegebenenfalls in ihr physiologisch annehmbares Salz umwandelt [A. Sitte u.a., «Chem. Ber.» 102, 615 (1969)].

Die Umsetzung erfolgt in einem Lösungsmittel und bei einer Temperatur von Raumtemperatur bis Siedetemperatur des verwendeten Lösungsmittels. Geeignete Lösungsmittel sind zum Beispiel Alkohole, wie Methanol oder Ethanol, Ether, wie Dioxan oder Tetrahydrofuran. Die Aufarbeitung erfolgt in an sich bekannter Weise, beispielsweise durch Einengen des Reaktionsgemisches und anschliessende Kristallisation.

Die erfindungsgemässen Verbindungen können mit geeigneten Säuren in ihre physiologisch annehmbaren Salze überführt werden. Die Umsetzung erfolgt in an sich bekannter Weise.

Geeignete Säuren sind anorganische und organische Säuren. Beispiele für anorganische Säuren sind Chlorwasserstoff-, Bromwasserstoff-, Schwefel-, Sulfamid-, Phosphor- und Salpetersäure. Beispiele für geeignete organische Säuren sind Malein-, Fumar-, Bernstein-, Oxal-, Äpfel-, Benzoe-, Methansulfon-, Ethandisulfon-, Benzolsulfon-, Essig-, Propion-, Wein-, Zitronen-, Camphersulfonsäure und ähnliche. Grundsätzlich sind alle in der Pharmazie geeigneten anorganischen und organischen Säuren für die Umwandlung in das physiologisch annehmbare Salz einsetzbar.

Bevorzugte Verbindungen sind 5-(6-Methylpyrid-3-ylmethyl)- 2-[4-[3-(1-piperidylmethyl) phenoxy]butyl]- pyrimidin-4-on, 5-(Pyrid-3-ylmethyl)- 2- [4-[3-(1-piperidylmethyl) phenoxy] butyl]-pyrimidin-4-on und 2-[3-[2-(1-Piperidylmethyl) thienyl-4-methylthio] propyl]-5- (pyrid-3-ylmethyl)- pyrimidin-4-on sowie die physiologisch annehmbaren Salze davon, wobei 5-(6-Methylpyrid-3-ylmethyl)- 2-[4-[3-(1-piperidylmethyl) phenoxy]butyl] -pyrimidin-4-on und die physiologisch annehmbaren Salze davon besonders bevorzugt werden.

Die erfindungsgemässen Verbindungen, vorzugsweise in der Form eines Salzes, können zur Verabreichung in jeder beliebigen Weise formuliert werden. Die Erfindung umfasst daher auch Arzneimittel, die mindestens eine erfindungsgemässe Verbindung zur Verwendung in der Human- oder Veterinärmedizin enthalten. Solche Arzneimittel können herkömmlicherweise unter Verwendung von einem oder mehreren pharmazeutisch annehmbaren Trägern oder Verdünnungsmitteln hergestellt werden.

Die erfindungsgemässen Verbindungen können daher für die orale, bukkale, topische, parenterale oder rektale Verabreichung formuliert werden, wobei die orale Verabreichung bevorzugt wird. Für die orale Verabreichung kann das Arzneimittel in Form von beispielsweise Tabletten, Kapseln, Pulvern, Lösungen, Sirups oder Suspensionen vorliegen, die unter Verwendung von annehmbaren Verdünnungsmitteln auf herkömmliche Weise hergestellt worden sind. Für die bukkale Verabreichung kann das Arzneimittel die Form von Tabletten oder Briefchen einnehmen, die in herkömmlicher Weise formuliert worden sind.

Die erfindungsgemässen Verbindungen können für die parenterale Verabreichung durch Bolusinjektion oder kontinuierliche Infusion formuliert werden. Formulierungen zur Injektion können in Dosiseinheitsform als Ampullen oder in Mehrfachdosenbehältern mit zugesetztem Konservierungsmittel vorliegen.

Die Arzneimittel können solche Formen, wie Suspensionen, Lösungen oder Emulsionen in öligen oder wässrigen Trägern, einnehmen, und sie können Formulierungshilfsmittel, wie Suspendierungs-, Stabilisierungs- und/oder Dispergierungsmittel, enthalten. Alternativ kann der Wirkstoff auch in Pulverform zur Rekonstitution mit einem geeigneten Träger, zum Beispiel sterilem, pyrogenfreiem Wasser, vor dem Gebrauch vorliegen.

Die erfindungsgemässen Verbindungen können auch für rektale Zubereitungen, zum Beispiel Suppositorien oder Retentionseinläufe, formuliert werden, die zum Beispiel herkömmliche Suppositoriengrundlagen, wie Kakaobutter oder andere Glyceride, enthalten.

Zur topischen Anwendung können die erfindungsgemässen Verbindungen als Salben, Cremes, Gels, Lotionen, Pulver oder Sprays in herkömmlicher Weise formuliert werden.

Für die orale Verabreichung ist eine geeignete Tagesdosis an erfindungsgemässen Verbindungen 1 bis 4 Dosen bis insgesamt 5 mg bis 1 g/Tag, vorzugsweise 5 bis 250 mg/Tag, je nach Zustand des Patienten. Im Einzelfall kann es gegebenenfalls erforderlich sein, von den genannten Mengen abzuweichen, und zwar in Abhängigkeit vom individuellen Verhalten gegenüber dem Wirkstoff bzw. der Art seiner Formulierung und dem Zeitpunkt bzw. Intervall, zu welchem die Verabreichung erfolgt. So gibt es zum Beispiel Fälle, wo mit weniger als der oben genannten Mindestmenge ausgekommen werden kann, während in anderen Fällen die genannte obere Grenze überschritten werden muss.

Die erfindungsgemässen Verbindungen zeichnen sich gegenüber anerkannt guten Arzneimitteln der gleichen Wirkungsrichtung durch eine Verbesserung der pharmakologischen Aktivitäten aus. Dies ergibt sich aus den Ergebnissen der im folgenden dargestellten pharmakologischen Vergleichsuntersuchungen.

Eine anerkannte Methode zur Bestimmung $H_2$-antagonistischer Wirksamkeit ist die Ermittlung der $pA_2$-Werte in vitro am isolierten Meerschweinchenvorhof (vgl. Ariens, Molecular Pharmacology, Band 1, Academic Press, New York, 1964).

| | $pA_2$-Werte | |
|---|---|---|
| Cimetidin: | 6,21 | Vergleich |
| Beispiel 1 | 7,66 | |

Andere Verbindungen der allgemeinen Formel I zeigen ähnliche pharmakologische Wirkungen.

Beispiel 1

5-(6-Methylpyrid-3-ylmethyl)-2-[4-[3-(1-piperidylmethyl) phenoxy]butyl]-pyrimidin-4-on.

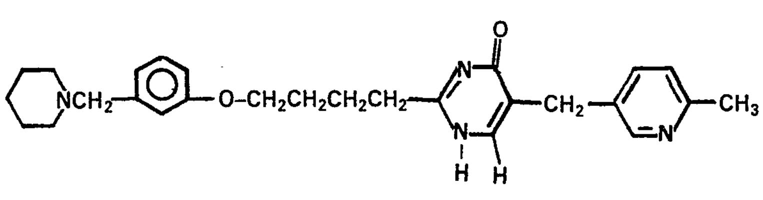

Eine Lösung von 2,2 g (10 mmol) 2-Formyl-3-(6-methyl-3-pyridyl)- propionsäureethylester in 20 ml Ethanol wird zur Lösung von 0,67 g (10 mmol) Natriummethylat in 20 ml Ethanol gegeben. Nach Zugabe von 3,53 g (12,2 mmol) 5-[3-(1-Piperidylmethyl) phenoxy] valeroamidin in 10 ml Ethanol wird die Mischung 48 Stunden bei Raumtemperatur gerührt, das Lösungsmittel im Vakuum eingeengt und das verbleibende Öl in 30 ml Wasser aufgenommen. Die wässrige Lösung wird dreimal mit 20 ml Chloroform extrahiert, die organische Phase über Natriumsulfat

getrocknet, filtriert und das Lösungsmittel im Vakuum entfernt. Das verbleibende Öl kristallisiert nach Anreiben.

Farblose Kristalle vom Schmelzpunkt 113 °C

$R_f$ = 0,3 ($CH_2Cl_2/CH_3OH$ 90:10)

Ausbeute: 2,85 g (64%) $C_{27}H_{34}N_4O_2$ (447)

| | |
|---|---|
| $^1$H-NMR-Spektrum: ($CDCl_3$, TMS als interner Standard) | δ = 1,20–2,44 (m) 14 H, |
| | 2,46 (s) (Py-$\underline{CH_3}$) 3 H, |
| | 2,71 (t) (–$\underline{CH_2}$–$CH_2$–$CH_2$–O) 2 H, |
| | 3,40 (s) ( N–$\underline{CH_2}$–) 2 H, |
| | 3,68 (s) (–$\underline{CH_2}$–Py) 2 H, |
| | 3,98 (t) (–O–$\underline{CH_2}$–) 2 H, |
| | 6,62–7,60 (m) (Aromaten-$\underline{H}$) 6 H, |
| | 7,82 (s) Py–$\underline{H}$) 1 H, |
| | 8,48 (s) (Py–$\underline{H}$) 1 H ppm. |

Beispiel 1a

Herstellung von 5-[3-(1-Piperidylmethyl) phenoxy] -valeroamidin-dihydrochlorid.

3,0 g (10 mmol) Methyl-5-[3-(1-piperidylmethyl) phenoxy] valeroimidat und 0,54 g (10 mmol) Ammoniumchlorid werden in 20 ml Methanol 24 Stunden bei Raumtemperatur gerührt. Nach weitgehendem Einengen werden 3,3 ml (~20 mmol) ethanolische Salzsäure zugesetzt und das entstandene Dihydrochlorid mit Ether gefällt.

Farblose Kristalle vom Schmelzpunkt 178–180 °C.

Rf = 0,6 ($CH_3OH$)

Ausbeute: 3,1 g (85%) $C_{17}H_{29}Cl_2N_3O$ (362)

| | | | |
|---|---|---|---|
| Ber.: | C 56,35 | H 8,07 | N 11,60 |
| Gef.: | C 56,44 | H 8,02 | N 11,53 |

Beispiel 1b

Herstellung von 2-Formyl-3- (6-methyl-3-pyridyl) -propionsäureethylester

a) Herstellung von 6-Methyl-3-pyridinaldehyd

80 g (0,37 mol) Natriumperjodat in 200 ml Wasser werden unter kräftigem Rühren zu 23 g (0,19 mol) 2-Methyl-5-vinyl- pyridin in 800 ml Ethylenglykoldimethylether getropft. Unter Kühlung mit Eis-Kochsalz-Mischung werden 1,0 g (0,04 mol) Osmiumtetroxid zugegeben und die Mischung 8 Stunden bei Raumtemperatur unter Stickstoffatmosphäre gerührt. Das nach Abdampfen des Lösungsmittel i.Vak. verbleibende Öl wird in wenig Wasser aufgenommen und dreimal mit Essigsäureethylester extrahiert. Nach dem Trocknen der organischen Phase über Natriumsulfat und Eindampfen i.Vak. verbleiben 20 g (87%) eines hellbraunen Öls, das sofort weiterverarbeitet wird.

b) Herstellung von 3-(6-Methyl-3-pyridyl)-acrylsäureethylester

Zu einer Lösung von 25 g (0,19 mol) Malonsäuremonoethylester in 50 ml Pyridin werden 1,5 ml Piperidin gegeben und dann 17 g (0,14 mol) 6-Methyl-3-pyridinaldehyd getropft. Die Mischung wird 5 Stunden unter Rückfluss gekocht und nach Abkühlen auf ein Gemisch aus Eis und konz. Salzsäure gegeben. Nach Ausschütteln mit Diethylether wird die organische Phase über Natriumsulfat getrocknet und im Vakuum eingedampft. Es werden 17,8 g (67%) der Titelverbindung als Öl erhalten, das ohne weitere Reinigung weiterverarbeitet wird.

c) Herstellung von 3-(6-Methyl-3-pyridyl)-propionsäureethylester

17,8 g (0,092 mol) 3-(6-Methyl-3-pyridyl)-acrylsäureethylester in 100 ml Ethanol werden in Gegenwart von 1,0 g Palladium/Aktivkohle (10% Pd) bei 40 °C und Atmosphärendruck hydriert. Nach beendeter Umsetzung wird filtriert und das Lösungsmittel im Vakuum eingeengt. Es verbleiben 16,2 g (90%) eines farblosen Öls.

d) Herstellung von 2-Formyl-3- (6-methyl-3-pyridyl)- propionsäureethylester

Zur Suspension von 5,2 g Natriumhydrid (80% auf Paraffinöl; 0,17 mol) in 30 ml Ethylenglykoldimethylether wird unter Eiskühlung langsam eine Mischung aus 16,0 g (0,083 mol) 3-(6-Methyl-3-pyridyl)- propionsäureethylester und 9,6 g (0,13 mol) Ameisensäureethylester getropft. Nach beendeter Zugabe wird die Lösung 8 Stunden bei Raumtemperatur gerührt. Nach Zusatz von 10 ml Essigsäureethylester und 200 ml Eiswasser wird die Mischung dreimal mit Diethylether extrahiert. Die wässrige Phase wird mit verdünnter Salzsäure auf pH 5,4 eingestellt und die ausfallenden Kristalle abgesaugt. Es werden 12,8 g (70%) hellbraune Kristalle vom Schmelzpunkt 141–142 °C erhalten.

Beispiel 2

5-(Pyrid-3- ylmethyl) -2-[4-[3-(1-piperidylmethyl) phenoxy] butyl]-pyrimidin-4-on

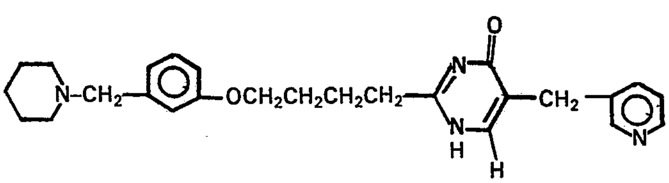

Das aus 2,9 g (10 mmol) 5-[3-(1-Piperidylmethyl)phenoxy] valeroamidin und 1,65 g (8 mmol) 2-Formyl-3- (3-pyridyl) propionsäureethylester analog zu Beispiel 1 erhaltene Öl wird zur Reinigung mit Methylenchlorid/Methanol 4:1 an Kieselgel chromatographiert. Nach Abdampfen des Laufmittels gibt die Hauptfraktion ein farbloses Öl, das mit n-Hexan kristallisiert wird.

Farblose Kristalle vom Schmelzpunkt 78–79 °C.

$R_f$ 0,4 ($CH_2Cl_2/CH_3OH$ 4:1)

Ausbeute: 2,1 g (60%) $C_{26}H_{32}N_4O_2$ (432,6)
$^1$H-NMR-Spektrum: ($CDCl_3$, TMS als interner Standard)
δ = 1,28–2,54 (m) 14 H,
2,73 (t)-$OCH_2CH_2CH_2\underline{CH_2}$–) 2 H,
3,45 (s) ( N–$\underline{CH_2}$–) 2 H,
3,73 (s) (Py–$\underline{CH_2}$–) 2 H,
3,98 (t) (–O–$\underline{CH_2}$–) 2 H,
6,70–7,70 (m) (Aromaten-$\underline{H}$) 6 H,
7,85 (s) 1 H,
8,41–8,65 (m) (Py–$\underline{H}$) 2 H,
12,20 (breit) (–N$\underline{H}$) 1 H ppm.

Beispiel 3

2-[3-[2- (1-Piperidylmethyl) thienyl- 4-methylthio] propyl]-5- (pyrid-3-ylmethyl)- pyrimidin-4-on

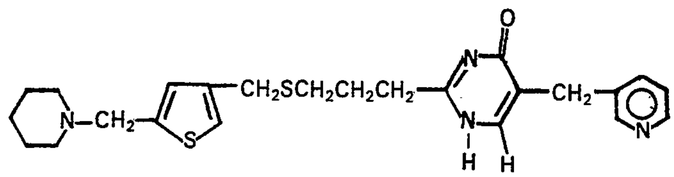

Zur Lösung von 3,1 g (10 mmol) 4-[2-(1-Piperidylmethyl) -thienyl-4-methylthio]- butyroamidin und 2,1 g (10 mmol) 2-Formyl-3- (3-pyridyl)- propionsäureethylester in 50 ml Ethanol werden 3 ml Triethylamin gegeben. Nach 48stündigem Rühren bei Raumtemperatur wird eine Stunde unter Rückfluss gekocht. Das nach Einengen des Lösungsmittels im Vakuum verbleibende Öl wird in 20 ml Wasser aufgenommen und die wässrige Phase dreimal mit 20 ml Chloroform extrahiert. Die organische Phase wird nach Trocknen über Natriumsulfat filtriert und eingedampft. Nach chromatographischer Reinigung mit Methylenchlorid/Methanol 9:1 an Kieselgel wird die Hauptfraktion eingedampft und mit Essigester angerieben.

Farblose Kristalle vom Schmelzpunkt 88 °C
$R_f$ = 0,3 ($CH_2Cl_2/CH_3OH$ 4:1)
Ausbeute: 2,0 g (44%) $C_{24}H_{30}N_4OS_2$ (454)
$^1$H-NMR-Spektrum: ($CDCl_3$, TMS als interner Standard)
δ = 1,37–2,90 (m) 16 H,
3,62 (s) ( N–$\underline{CH_2}$–, Py$\underline{CH_2}$, 2 H
3,64 (s) –$\underline{CH_2}$S–) 2 H,
3,75 (s) 2 H,
6,87 (s) (Thiophen-$\underline{H}$) 1 H,
6,95 (s) (Thiophen-$\underline{H}$) 1 H,
7,12–7,73 (m) (Py–$\underline{H}$) 2 H,
7,84 (s) 1 H,
8,43–8,67 (m) (Py–$\underline{H}$) 2 H,
12,6 (breit) (–N$\underline{H}$) 1 H ppm.

**Patentansprüche für die Vertragsstaaten BE, CH, DE, FR, GB, IT, LI, LU, NL und SE**

1. Pyridin-Pyrimidinon-Derivate der allgemeinen Formel I:

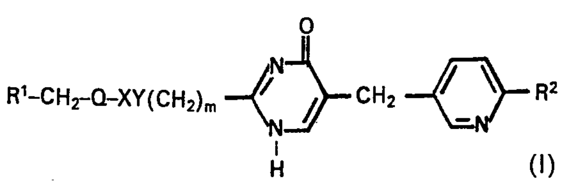

(I)

in der

$R^1$ für Pyrrolidino oder Piperidino und
$R^2$ für Wasserstoff oder Methyl steht,
Q für meta-Phenylen oder 2,4-Thiophendiyl stehen,

X im Fall, dass Q die Bedeutung m-Phenylen hat, für Sauerstoff, Y für eine Einfachbindung steht und m den Wert 4 hat,

X im Fall, dass Q die Bedeutung 2,4-Thiophendiyl hat, für Methylen, Y für Schwefel steht und m den Wert 3 hat, sowie die physiologisch annehmbaren Salze davon.

2. 5-(6-Methylpyrid-3-ylmethyl) -2- [4-[3-(1-piperidylmethyl) phenoxy]butyl] -pyrimidin-4-on und die physiologisch annehmbaren Salze davon.

3. 5-(Pyrid-3-ylmethyl) -2- [4-[3-(1-piperidylmethyl) phenoxy]butyl] -pyrimidin-4-on und die physiologisch annehmbaren Salze davon.

4. 2-[3-[2-(1-Piperidylmethyl) thienyl-4-methylthio] -propyl] -5- (pyrid-3-ylmethyl)- pyrimidin-4-on und die physiologisch annehmbaren Salze davon.

5. Verfahren zur Herstellung der Verbindungen nach Anspruch 1, dadurch gekennzeichnet, dass man ein Amidin der allgemeinen Formel II:

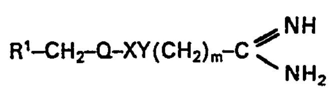

(II)

worin $R^1$, Q, X, Y und m die in Anspruch 1 angegebenen Bedeutungen haben, in an sich bekannter Weise in basenkatalysierter Reaktion mit einem Pyridinderivat der allgemeinen Formel III:

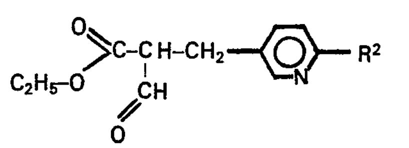

(III)

worin $R^2$ die in Anspruch 1 angegebene Bedeutung hat, umsetzt und die erhaltene Verbindung gegebenenfalls in ihr physiologisch annehmbares Salz umwandelt.

6. Arzneimittel, enthaltend eine Verbindung nach Anspruch 1 und mindestens einen inerten, pharmazeutisch annehmbaren Träger oder ein

inertes, pharmazeutisch annehmbares Verdünnungsmittel.

**Patentansprüche für den Vertragsstaat AT**

1. Verfahren zur Herstellung von Pyridin-Pyrimidinon-Derivaten der allgemeinen Formel I:

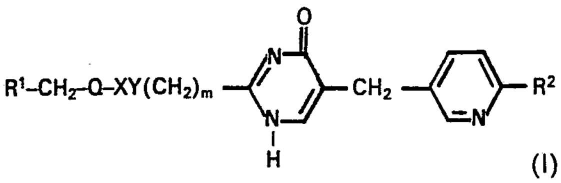

(I)

in der

$R^1$ für Pyrrolidino oder Piperidino und

$R^2$ für Wasserstoff oder Methyl stehen,

Q für meta-Phenylen oder 2,4-Thiophendiyl steht,

X im Fall, dass Q die Bedeutung m-Phenylen hat, für Sauerstoff, Y für eine Einfachbindung steht und m den Wert 4 hat,

X im Fall, dass Q die Bedeutung 2,4-Thiophendiyl hat, für Methylen, Y für Schwefel steht und m den Wert 3 hat, sowie der physiologisch annehmbaren Salze davon, dadurch gekennzeichnet, dass man ein Amidin der allgemeinen Formel II:

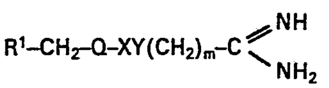

(II)

worin $R^1$, Q, X, Y und m die oben angegebenen Bedeutungen haben, in an sich bekannter Weise in basenkatalysierter Reaktion mit einem Pyridinderivat der allgemeinen Formel III:

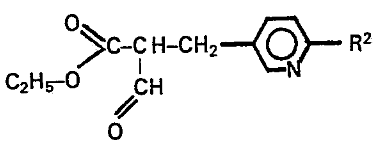

(III)

worin $R^2$ die oben angegebene Bedeutung hat, umsetzt und die erhaltene Verbindung gegebenenfalls in ihr physiologisch annehmbares Salz umwandelt.

2. Verfahren nach Anspruch 1 zur Herstellung von 5-(6-Methylpyrid-3-ylmethyl)-2-[4-[3-(1-piperidylmethyl) phenoxy]-butyl]-pyrimidin-4-on und der physiologisch annehmbaren Salze davon.

3. Verfahren nach Anspruch 1 zur Herstellung von 5-(Pyrid-3-ylmethyl) -2- [4-[3-(1-piperidylmethyl) phenoxy]butyl]-pyrimidin-4-on und der physiologisch annehmbaren Salze davon.

4. Verfahren nach Anspruch 1 zur Herstellung von 2-[3-[2-(1-Piperidylmethyl) thienyl-4-methylthio] propyl] -5- (pyrid-3-ylmethyl)-pyrimidin-4-on und der physiologisch annehmbaren Salze davon.

**Revendications pour les Etats contractants: BE, CH, DE, FR, GB, IT, LI, LU, NL, SE**

1. Dérivés de pyridine-pyrimidinone de formule générale I:

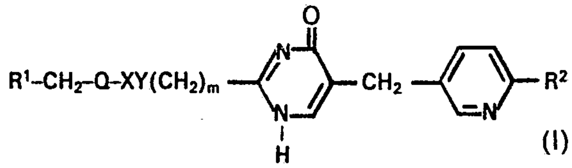

(I)

dans laquelle:

$R^1$ représente un groupe pyrrolidino ou pipéridino et

$R^2$ représente l'hydrogène ou un groupe méthyle,

Q représente un groupe méta-phénylène ou 2,4-thiophènediyle,

X, dans le cas où Q a la signification m-phénylène, représente l'oxygène, Y représente une liaison simple et m a la valeur 4,

X, dans le cas où Q a la signification 2,4-thiophènediyle, représente un groupe méthylène, Y représente le soufre et m a la valeur 3, ainsi que leurs sels physiologiquement acceptables,

2. La 5-(6-méthylpyrid-3-ylméthyl) -2- [4-[3-(1-pipéridylméthyl)- phénoxy] butyl]-pyrimidin-4-one et ses sels physiologiquement acceptables.

3. Le 5-(pyrid-3-ylméthyl) -2- [4-]3-(1-pipéridylméthyl) phénoxy] butyl]- pyrimidin-4-one et ses sels physicologiquement acceptables.

4. La 2-[3-[2- (1-pipéridylméthyl) thiényl-4-méthylthio]- propyl] -5- (pyrid-3-ylméthyl) -pyrimidin-4-one et ses sels physiologiquement acceptables.

5. Procédé pour la préparation des composés selon la revendication 1, caractérisé en ce que l'on fait réagir une amidine de formule générale II:

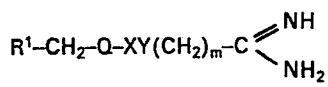

(II)

dans laquelle $R^1$, Q, X, Y et m ont les significations indiquées dans la Revendication 1, suivant une réaction catalysée par une base de manière connue en soi avec un dérivé de pyridine de formule générale III:

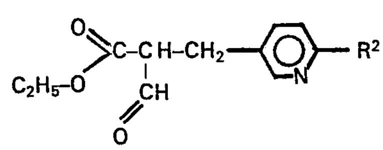

(III)

dans laquelle $R^2$ a la signification indiquée dans la Revendication 1, et on convertit le cas échéant le composé obtenu en son sel physiologiquement acceptable.

6. Médicament contenant un composé selon la Revendication 1 et au moins un véhicule inerte, pharmaceutiquement acceptable ou un diluant inerte, pharmaceutiquement acceptable.

**Revendications pour l'Etat contractant: AT**

1. Procédé pour la préparation de dérivés de pyridine-pyrimidinone de formule générale I:

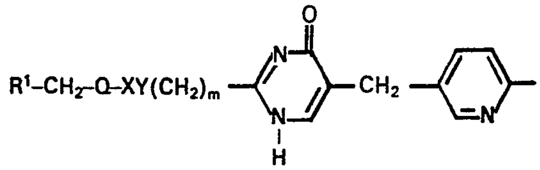

(I)

dans laquelle:

$R^1$ représente un groupe pyrrolidino ou pipéridino et

$R^2$ représente l'hydrogène ou un groupe méthyle,

Q représente un groupe méta-phénylène ou 2,4-thiophènediyle,

X, dans le cas où Q a la signification m-phénylène, représente l'oxygène, Y représente une liaison simple et m a la valeur 4,

X, dans le cas où Q a la signification 2,4-thiophènediyle, représente un groupe méthylène, Y représente le soufre et m a la valeur 3, ainsi que leurs sels physiologiquement acceptables, caractérisé en ce que l'on fait réagir une amidine de formule générale II:

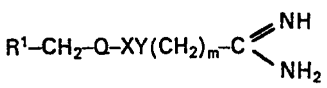

(II)

dans laquelle $R^1$, Q, X, Y et m ont les significations indiquées ci-dessus, suivant une réaction catalysée par une base de manière connue en soi avec un dérivé de pyridine de formule générale III:

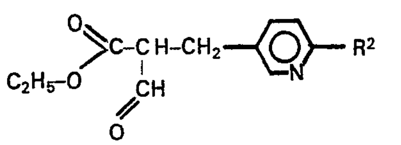

(III)

dans laquelle $R^2$ a la signification indiquée ci-dessus, et on convertit le cas échéant le composé obtenu en son sel physiologiquement acceptable.

2. Procédé selon la Revendication 1 pour la préparation de la 5-(6-méthylpyrid-3-ylméthyl) -2- [4-[3-(1-pipéridylméthyl) phénoxy]butyl]-pyrimidin-4-one et ses sels physiologiquement acceptables.

3. Procédé selon la revendication 1 pour la préparation de la 5-(pyrid-3-ylméthyl) -2- [4-[3-(1-pipéridylméthyl) phénoxy] butyl] -pyrimidin-4-one et ses sels physiologiquement acceptables.

4. Procédé selon la Revendication 1 pour la préparation de la 2-[3-[2-(1-pipéridylméthyl) thiényl-4-méthylthio] -propyl] -5- (pyrid-3-ylméthyl) -pyrimidin-4-one et ses sels physiologiquement acceptables.

**Claims for the Contracting States BE, CH, DE, FR, GB, IT, LI, LU, NL and SE**

1. Pyridine-pyrimidinone derivatives corresponding to the general formula I

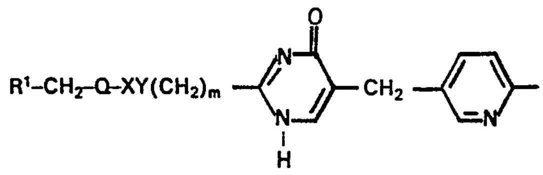

(I)

wherein

$R^1$ stands for pyrrolidino or piperidino and

$R^2$ stands for hydrogen or methyl and

Q stands for meta-phenylene or 2,4-thiophendiyl, and when Q stands for m-phenylene then X denotes oxygen,

Y denotes a single bond and m has the value 4, and when Q stands for 2,4-thiophendiyl then X denotes methylene,

Y denotes sulphur and m has the value 3, and the physiologically acceptable salts thereof.

2. 5-(6-Methylpyrid-3-ylmethyl)- 2-[4-[3-(1-piperidylmethyl)- phenoxy]butyl]-pyrimidin-4-one and the physiologically acceptable salts thereof.

3. 5-(Pyrid-3-ylmethyl) -2- [4-[3-(1-piperidylmethyl) phenoxy]butyl]-pyrimidin-4-one and the physiologically acceptable salts thereof.

4. 2-[3-[2-(1-Piperidylmethyl) thienyl-4-methylthio]-propyl] -5-(pyrid-3-ylmethyl)- pyrimidin-4-one and the physiologically acceptable salts thereof.

5. Process for the preparation of compounds according to claim 1, characterised in that an amidine corresponding to the general formula II

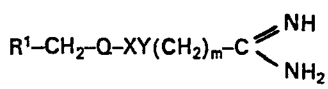

(II)

wherein $R^1$, Q, X, Y and m have the meanings indicated in claim 1 are reacted in known manner in a base-catalysed reaction with a pyridine derivative corresponding to the general formula III

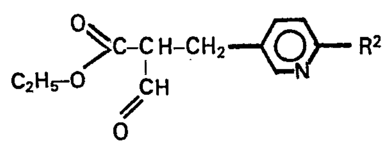

(III)

wherein $R^2$ has the meaning indicated in claim 1 and the compound obtained is optionally converted into a physiologically acceptable salt thereof.

6. Pharmaceutical preparation containing a compound according to claim 1 and at least one inert, pharmaceutically acceptable carrier or an inert, pharmaceutically acceptable diluent.

**Claims for the Contracting State: AT**

1. Process for the preparation of pyridine-pyrimidinone derivatives corresponding to the general formula I

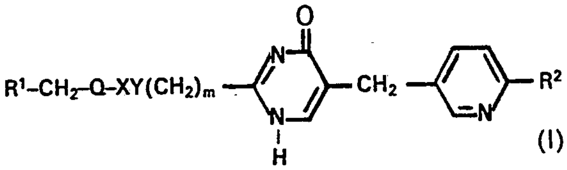

(I)

wherein

$R^1$ stands for pyrrolidino or piperidino and

$R^2$ stands for hydrogen or methyl,

Q stands for meta-phenylene or 2,4-thiophendiyl, and when Q stands for m-phenylene then X denotes oxygen,

Y denotes a single bond and m has the value 4, and when Q stands for 2,4-thiophendiyl then X denotes methylene,

Y denotes sulphur and m has the value 3, and the physiologically acceptable salts thereof, characterised in that an amidine corresponding to the general formula II

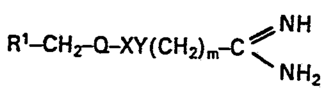

wherein $R^1$, Q, X, Y and m have the meanings indicated above is reacted in known manner in a base-catalysed reaction, with a pyridine derivative corresponding to the general formula III

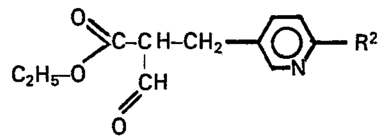

(III)

wherein $R^2$ has the meaning indicated above, and the compound obtained is optionally converted into a physiologically acceptable salt thereof.

2. Process according to claim 1 for the preparation of 5-(6-methylpyrid-3-ylmethyl) -2-[4-[3-(1-piperidylmethyl)- phenoxy]-butyl]-pyrimidin-4-one and the physiologically acceptable salts thereof.

3. Process according to claim 1 for the preparation of 5-(5-pyrid-3-ylmethyl) -2- [4-[3-(1-piperidylmethyl)- phenoxy]butyl]-pyrimidin-4-one and the physiologically acceptable salts thereof.

4. Process according to claim 1 for the preparation of 2-[3-[2-(1-piperidylmethyl) thienyl-4-methylthio]propyl]-5- (pyrid-3-ylmethyl)- pyrimidin-4-one and the physiologically acceptable salts thereof.